# EUROPEAN PATENT SPECIFICATION

(11) **EP 0 812 843 B1**
(45) Date of publication and mention of the grant of the patent: **30.01.2002**
(21) Application number: 97303989.4
(22) Date of filing: 09.06.1997
(51) Int. Cl.: C07D 471/04, C07D 401/04, C07C 251/32

(54) **Process for the preparation of 2,3-pyridinedicarboximides**
Verfahren zur Herstellung von 2,3-Pyridindicarbonsäureimide
Procédé pour la préparation de 2,3-pyridinedicarboximides

(30) Priority: 10.06.1996 US 661277
(43) Date of publication of application: 17.12.1997
(73) Proprietor: BASF AKTIENGESELLSCHAFT, 67056 Ludwigshafen (DE)
(72) Inventor: Kremer, Kenneth Alfred Martin, Lawrenceville, New Jersey 08648 (US); Wu, Wen-Xue, Lawrenceville, New Jersey 08648 (US); Maulding, Donald Roy, Somerville, New Jersey 08876 (US)
(74) Representative: Köster, Reinhold, Dr.

(56) References cited:
- EP-A- 0 041 623
- EP-A- 0 161 221
- EP-A- 0 172 140
- EP-A- 0 205 879
- EP-A- 0 308 084
- SYLVIA J. ALLCOCK ET AL: "Intramolecular and intermolecular Diels-Alder reactions of acylhydrazones derived from methacrolein and ethylacrolein" TETRAHEDRON., vol. 47, no. 48, 9 December 1991, OXFORD GB, pages 10053-10064, XP002056033
- ADRIAN WALDNER: "[4+2]-Cycloadditionen von alpha,beta-ungesättigten Hydrazonen" HELVETICA CHIMICA ACTA., vol. 71, no. 2, 16 March 1988, BASEL CH, pages 486-492, XP002055963
- J. MARCH: 'Advanced Organic Chemistry', 1985, WILEY

## Description

### BACKGROUND OF THE INVENTION

2,3-Pyridinedicarboximides are useful as intermediates in the preparation of herbicidal 2-(2-imidazolin-2-yl)nicotinic acids, esters and salts. Methods for the preparation of 2,3-pyridinedicarboximides are known in the art (see, e.g., U.S. 4,748,244; U.S. 4,754,033 and EP 308,084-A1). However, the methods described in those patents and patent application are not entirely satisfactory for the commercial manufacture of 2,3-pyridinedicarboximides.

It is, therefore, an object of the present invention to provide an effective and efficient process for the preparation of 2,3-pyridinedicarboximides.

It is also an object of the present invention to provide a compound which is useful in the process of this invention.

These and other objects and features of the present invention will become more apparent from the detailed description thereof set forth below.

### SUMMARY OF THE INVENTION

The present invention provides an effective and efficient process for the preparation of a 2,3-pyridine-dicarboximide having the structural formula I 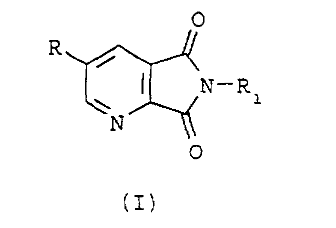 wherein
- R: is hydrogen, C₁-C₆alkyl or C₁-C₆alkoxymethyl;
- R₁: is hydrogen, C₁-C₆alkyl, C(O)R₂,
phenyl optionally substituted with any combination of from one to four halogen, C₁-C₄alkyl, C₁-C₄alkoxy, nitro or cyano groups,
benzyl optionally substituted on the phenyl ring with any combination of from one to four halogen, C₁-C₄alkyl, C₁-C₄alkoxy, nitro or cyano groups, or 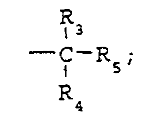
- R₂: is C₁-C₆alkyl, benzyl or
phenyl optionally substituted with any combination of from one to four halogen, C₁-C₄alkyl, C₁-C₄alkoxy, nitro or cyano groups;
- R₃ and R₄: are each independently C₁-C₄alkyl; and
- R₅: is cyano or CONH₂,
which process comprises reacting an oxime or hydrazone having the structural formula II 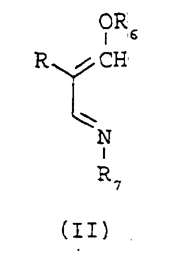 wherein
- R: is as described above;
- R₆: is C₁-C₆alkyl;
- R₇: is OR₈ or NR₉R₁₀;
- R₈: is hydrogen, C₁-C₆alkyl, C(O)R₁₁,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups;
- R₁₁: is C₁-C₆alkyl, OR₁₂, NR₁₂R₁₃, benzyl or
phenyl optionally substituted with any combination of from one to four halogen, C₁-C₄alkyl, C₁-C₄alkoxy, nitro or cyano groups;
- R₁₂ and R₁₃: are each independently hydrogen, C₁-C₆alkyl, benzyl or
phenyl optionally substituted with any combination of from one to four halogen, C₁-C₄alkyl, C₁-C₄alkoxy, nitro or cyano groups; and
- R₉ and R₁₀: are each independently hydrogen, C₁-C₆alkyl, benzyl or
phenyl optionally substituted with any combination of from one to four halogen, C₁-C₄alkyl, C₁-C₄alkoxy, nitro or cyano groups,
with a maleimide having the structural formula III 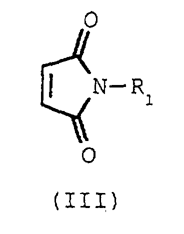 wherein R₁ is as described above.

This invention also relates to the formula II oximes described hereinabove.

In one preferred embodiment of the present invention, an oxime or hydrazone represented by formula II is reacted with a maleimide represented by formula III, preferably at a temperature above about 20°C e.g. in a temperature range of about 20°C to 160 °C, in the presence of a solvent.

Advantageously, it has now been found that 2,3-pyridinedicarboximides may be obtained in high yield and/or high purity by the effective and efficient process of the present invention.

The 2,3-pyridinedicarboximides may be isolated by diluting the reaction mixture with water and filtering the formula I product from the aqueous mixture. The product formula I compounds may also be isolated by concentrating the reaction mixture *in vacuo* and filtering the formula I product from the concentrated mixture. Alternatively, the reaction mixture may be integrated into the process used to prepare the final herbicidal agent without isolating the formula I compound.

Exemplary of halogen hereinabove are fluorine, chlorine, bromine and iodine. When used herein as a group or part of a group the term alkyl includes straight or branched chain alkyl groups such as methyl, ethyl, n-propyl, isopropyl, n-butyl, isobutyl, s-butyl and t-butyl.

In another embodiment of the present invention, a Lewis acid is present. Preferably, the Lewis acid is present in an amount up to about one molar equivalent relative to the formula II compound when R₈ is hydrogen. Lewis acids suitable for use in the present invention include any conventional Lewis acids. Preferred Lewis acids include aluminum chloride and titanium(IV) chloride.

Solvents suitable for use in the process of the present invention preferably have a boiling point of at least about 60 °C and include aromatic hydrocarbons such as toluene, xylenes, mesitylene and mixtures thereof; halogenated aromatic hydrocarbons such as mono- and dihalobenzenes and mixtures thereof; polynuclear aromatic hydrocarbons such as naphthalene, alkylnaphthalenes and mixtures thereof; ethers such as tetrahydrofuran and mixtures thereof; glycols such as 1,2-diethoxyethane and mixtures thereof; an alkanoic acid such as acetic acid, propionic acid and mixtures thereof; an alkanoic acid/water mixture such as an acetic acid/water mixture; acetonitrile; an acetonitrile/water mixture; and mixtures thereof. Preferred solvents include toluene, xylenes, mesitylene, acetonitrile, an acetonitrile/water mixture, acetic acid and mixtures thereof with toluene and acetonitrile being more preferred.

In another preferred embodiment of the present invention, oximes of formula II wherein R₇ is OR₈ are reacted with maleimides of formula III preferably at a temperature above about 60°C e.g. at a temperature range of about 60 °C to 160 °C, more preferably about 75 °C to 135 °C. And hydrazones of formula II wherein R₇ is NR₉R₁₀ are reacted with maleimides of formula III preferably at a temperature above about 20°C e.g. at a temperature range of about 20 °C to 160 °C, more preferably about 20 °C to 135 °C.

In a further preferred embodiment of the present invention, a base is present when R is C₁-C₆alkoxymethyl. The base is used to reduce the amount of 5-methyl-2,3-pyridinedicarboximides which are produced as undesirable by-products when R is C₁-C₆alkoxymethyl.

Bases suitable for use in the present invention include, but are not limited to, tri(C₂-C₄alkyl)amines such as triethylamine, N,N-diethylisopropylamine, N,N-diisopropylethylamine and the like, alkali metal acetates such as sodium acetate, potassium acetate and the like, and mixtures thereof. Preferred bases include triethylamine, sodium acetate and potassium acetate. The base is preferably present in an amount of at least about one molar equivalent relative to the formula II compound.

In a further embodiment of the present invention, a phase transfer catalyst is present when the base is present. Preferably, the phase transfer catalyst is present when the alkali metal acetate is present. Phase transfer catalysts suitable for use in the present invention include any conventional phase transfer catalysts. Preferred phase transfer catalysts include crown ethers such as 18-crown-6 and 15-crown-5.

In a preferred process of the present invention,
- R: is hydrogen, C₁-C₄alkyl or C₁-C₄alkoxymethyl;
- R₁: is hydrogen, C₁-C₄alkyl,
phenyl optionally substituted with any combination of from one to four halogen, C₁-C₄alkyl, C₁-C₄alkoxy, nitro or cyano groups, or 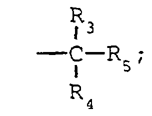 and/or
- R₃ and R₄: are each independently C₁-C₄alkyl; and/or
- R₅: is cyano or CONH₂; and/or
- R₆: is C₁-C₄alkyl;
- R₇: is OR₈; and/or
- R₈: is hydrogen or C₁-C₆alkyl.

In a more preferred process of the present invention,
- R: is hydrogen, methyl, ethyl or methoxymethyl; and/or
- R₁: is methyl, phenyl or 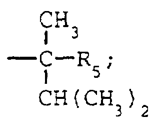 and/or
- R₅: is cyano or CONH₂; and/or
- R₆: is methyl or ethyl; and/or
- R₇: is OR₈; and/or
- R₈: is hydrogen or methyl.
Formula II oximes wherein
- R₇: is OR₈; and/or
- R₈: is hydrogen, C₁-C₆alkyl,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups,
may be prepared by reacting a 3-alkoxy-2-propenal of formula IV with a substituted hydroxylamine of formula V optionally in the presence of a base. The reaction scheme is shown below in Flow Diagram I.

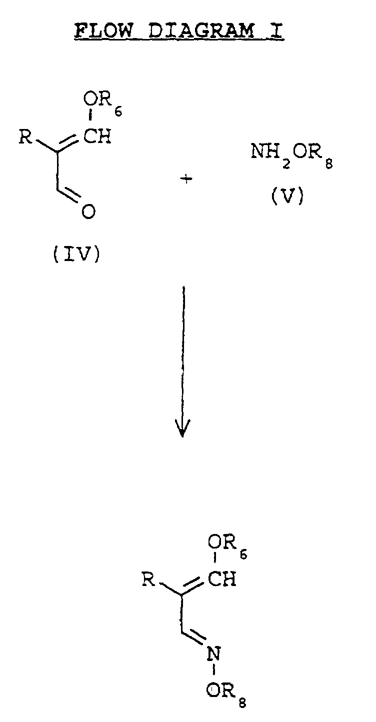

Alternatively, oximes of formula II wherein R₈ is C₁-C₆alkyl may be prepared by reacting a formula II compound wherein R₈ is hydrogen with a dialkyl sulfate of formula VI in the presence of a base such as sodium hydroxide or an alkali metal alkoxide. The reaction scheme is shown in Flow Diagram II.

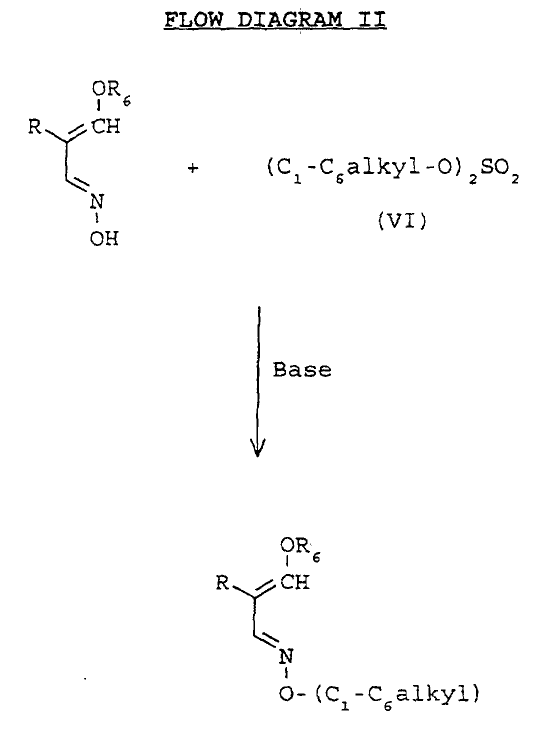

Formula II oximes wherein R₈ is C(O)R₁₁ may be prepared by reacting a formula II compound wherein R₈ is hydrogen with an acid chloride of formula VII or an anhydride of formula VIII as shown in Flow Diagram III.

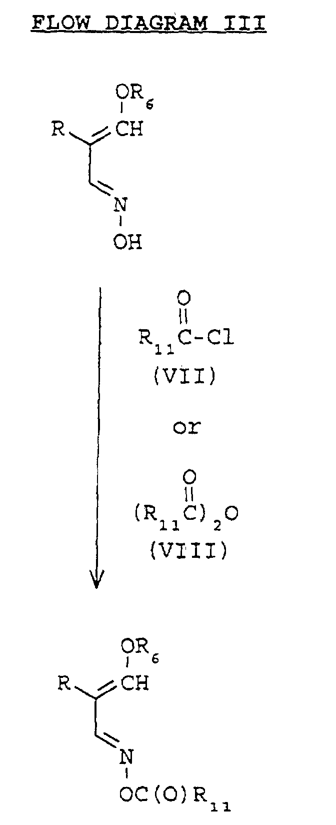

Formula II hydrazones may be prepared by reacting a 3-alkoxy-2-propenal of formula IV with a hydrazine of formula IX optionally in the presence of an acid catalyst such as acetic acid. The reaction scheme is shown in Flow Diagram IV.

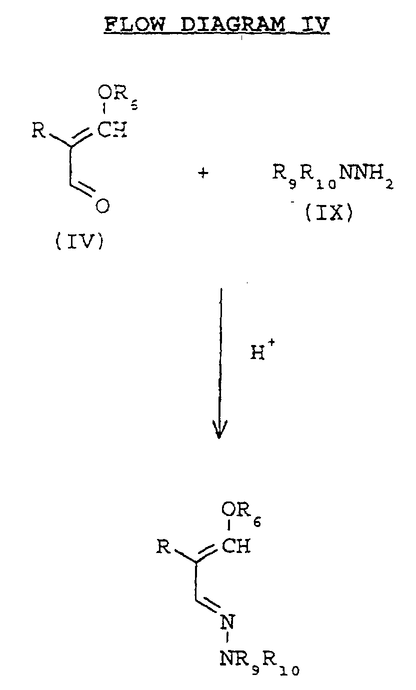

3-Alkoxy-2-propenal compounds of formula IV may be prepared according to the procedures described by E. Breitmaier, *et al* in Synthesis, pages 1-9 (1987).
Maleimide compounds of formula III are known in the art and may be prepared according to the procedures described by M. Cava, *et al* in Organic Synthesis, 41, pages 93 (1961).

Alternatively, formula IV compounds wherein R is methoxymethyl may be prepared by reacting a 3-(dialkylamino)-2-propenal of formula X with formaldehyde and methanol in the presence of a mineral acid such as sulfuric acid to form a 3-(dialkylamino)-2-(methoxymethyl)-2-propenal of formula XI, and reacting the formula XI compound with a base such as an alkali metal hydroxide and a dialkyl sulfate of formula VI. The reaction scheme is shown in Flow Diagram V.

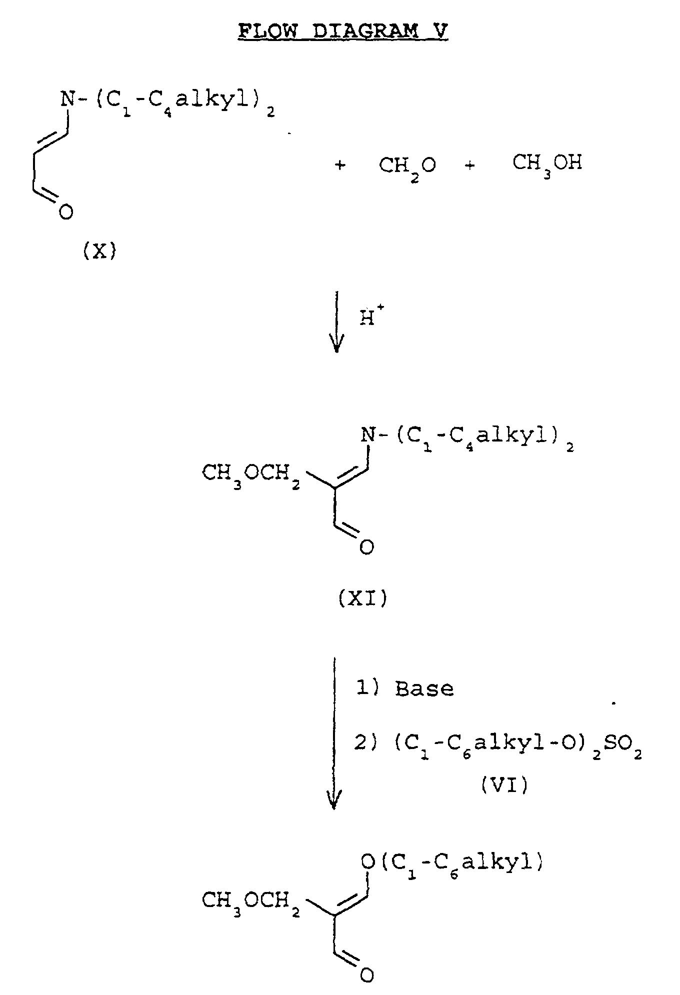

The present invention also provides a process for the preparation of a herbicidal 5-(alkoxymethyl)-2-(2-imidazolin-2-yl)-nicotinic acid, ester and salt compound having the formula 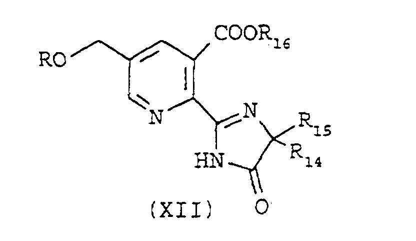 wherein
- R: is as defined above;
- R₁₄: is C₁-C₄ alkyl;
- R₁₅: is C₁-C₄ alkyl, C₃-C₆ cycloalkyl or R₁₄ and R₁₅ when taken together with the atom to which they are attached, represent a C₃-C₆ cycloalkyl group optionally substituted with methyl and
- R₁₆: is hydrogen, diloweralkylimino,
- C₁-C₁₂: alkyl optionally substituted with one of the following groups: C₁-C₃ alkoxy, halogen, hydroxy, C₃-C₆ cycloalkyl, benzyloxy, furyl, phenyl, halophenyl, lower alkylphenyl, lower alkoxyphenyl, nitrophenyl, carboxyl, loweralkoxycarbonyl, cyano or triloweralkylammonium;
- C₃-C₁₂: alkenyl optionally substituted with one of the following groups: C₁-C₃ alkoxy, phenyl, halogen or loweralkoxycarbonyl or with two C₁-C₃ alkoxy groups or two halogen groups;
- C₃-C₆: cycloalkyl optionally substituted with one or two C₁-C₃ alkyl groups; or
a cation preferably selected from the group consisting of alkali metals, alkaline earth metals, manganese, copper, iron, zinc, cobalt, lead, silver, nickel, ammonium and organic ammonium;
which process comprises:
(a) preparing a compound having the formula I 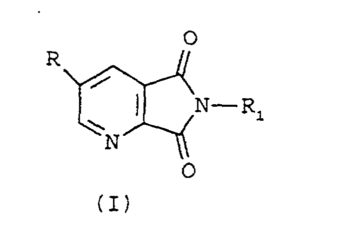 wherein R and R₁ are as defined above by a process as defined above; and
(b) converting the compound having formula I into the compound having the formula XII.

The term "lower" as used above in relation to alkyl and alkoxy groups means that the alkyl or alkoxy group contains 1 to 6, preferably 1 to 4, carbon atoms.

The conversion of the compound having formula I into the compound having formula XII may be carried out in a variety of ways. One may plan routes by combining reactions known for the conversion of one carboxylic acid derivative into another.

Methods that may be used to create the imidazolinone herbicides are illustrated in the book "The Imidazolinone Herbicides" edited by D.L. Shaner and S.L. O'Connor, published 1991 by CRC Press, Boca Raton, Florida with particular reference to Chapter 2 entitled "Synthesis of the Imidazolinone Herbicides", pages 8-14 and the references cited therein. The following patent literature references also illustrate the methods that may be used to convert the carboxylic acid derivatives into imidazolinone final products:
U.S. Patent Nos. 5,371,229; 5,334,576; 5,250,694;
5,276,157; 5,110,930; 5,122,608; 5,206,368;
4,925,944; 4,921,961; 4,959,476; 5,103,009;
4,816,588; 4,748,244; 4,754,033; 4,757,146;
4,798,619; 4,766,218; 5,001,254; 5,021,078;
4,723,011; 4,709,036; 4,658,030; 4,608,079;
4,719,303; 4,562,257; 4,518,780; 4,474,962;
4,623,726; 4,750,978; 4,638,068; 4,439,607;
4,459,408; 4,459,409; 4,460,776; 4,125,727 and
4,758,667, and European Patent Application Nos. EP-A-0-041,623 and EP-A-0-308,084.

In order to facilitate a further understanding of the invention, the following examples are presented primarily for the purpose of illustrating more specific details thereof. The invention should not be deemed limited by the examples as the full scope of the invention is defined in the claims.

### EXAMPLE 1

### Preparation of the Oxime of 3-ethoxy-2-methyl-2-propen-1-one, (E)- and (Z)-

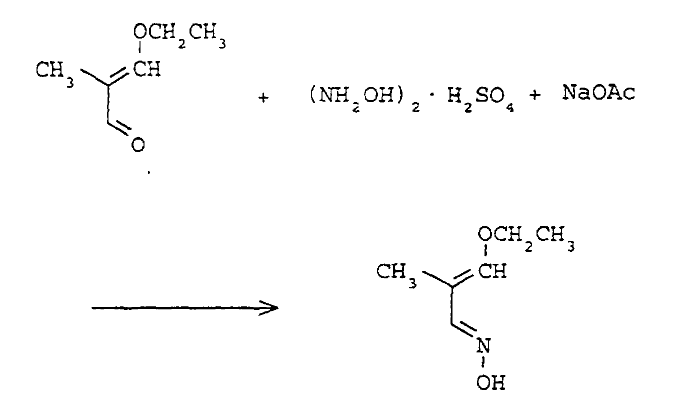

3-Ethoxy-2-methyl-2-propenal, (E)- and (Z)- (30.0 g, 0.25 mol) is added dropwise to a mixture of hydroxylamine sulfate (33.0 g, 0.2 mol) and sodium acetate (33.4 g, 0.4 mol) in water (200 g). The resultant reaction mixture is stirred overnight and filtered co obtain a solid. The solid is washed with water and dried to give the title product as a white solid (23.2 g, mp 78 °C, 71% yield).

Using essentially the same procedure, but substituting methoxylamine hydrochloride for hydroxylamine sulfate, the O-methyloxime of 3-ethoxy-2-methyl-2-propen-1-one, (E)- and (Z)- is obtained as a yellow oil.

### EXAMPLE 2

### Preparation of the O-methyloxime of 3-ethoxy-2-methyl-2-propen-1-one, (E)- and (Z) -

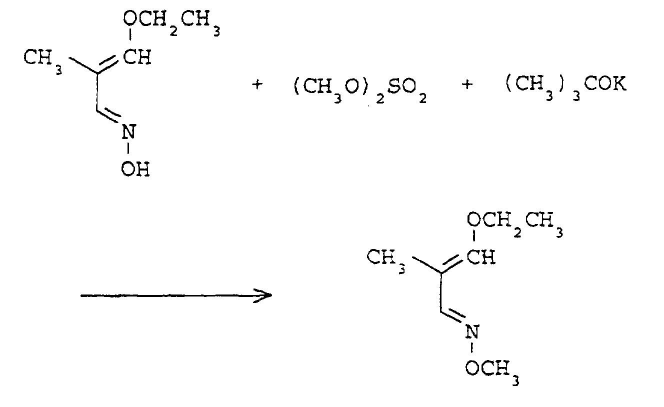

A mixture of the oxime of 3-ethoxy-2-methyl-2-propen-1-one, (E)- and (Z)- (0.5 g, 3.87 mmol) and potassium *tert*-butoxide (0.48 g, 4.2 mmol) in tetrahydrofuran is stirred for ten minutes at 10 °C, treated dropwise with dimethyl sulfate (0.59 g, 4.6 mmol), stirred for two hours and filtered. The resultant filtrate is concentrated in vacuo to give the title product as a yellow oil (0.74 g, 100% yield).

### EXAMPLE 3

### Preparation of 5-Methyl-N-phenyl-2,3-pyridine-dicarboximide

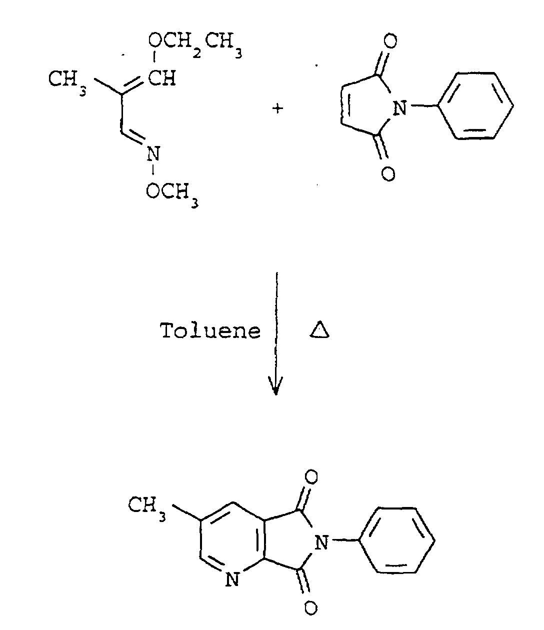

A solution of N-phenylmaleimide (1.69 g, 9.8 mmol) in toluene (16 g) is refluxed for 24 hours. During the reflux period, the O-methyloxime of 3-ethoxy-2-methyl-2-propen-1-one, (E)- and (Z)- (1.57 g, 11 mmol) is added portionwise to the reaction mixture. The final reaction mixture is then concentrated in vacuo to give the title product as a orange solid (1.2 g, 52% yield).

### EXAMPLES 4-7

Using essentially the same procedure as described in Example 3, but substituting the oxime of 3-ethoxy-2-methyl-2-propen-1-one, (E)- and (Z)- for the O-methyloxime of 3-ethoxy-2-methyl-2-propen-1-one, (E)- and (Z)-, 5-methyl-N-phenyl-2,3-pyridinedicarboximide is produced in the yields shown in Table I.

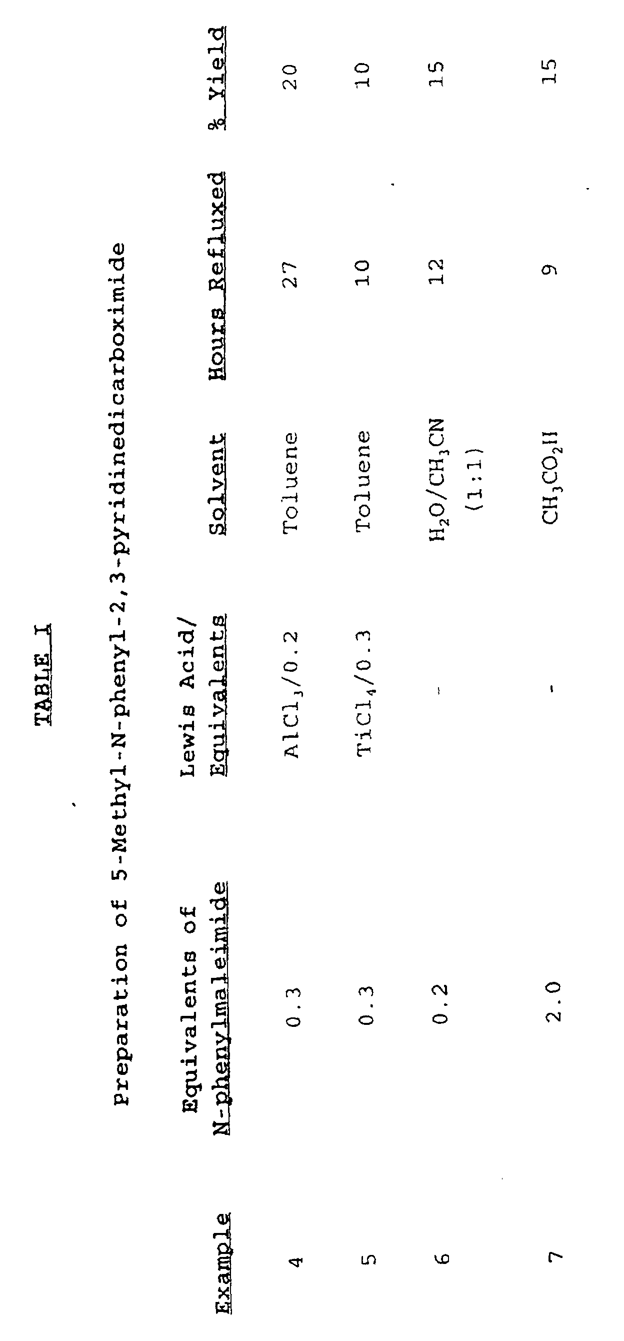

### EXAMPLE 8

### Preparation of 3-(Dimethylamino)-2-(methoxymethyl)-2-propenal, (E)- and (Z)-

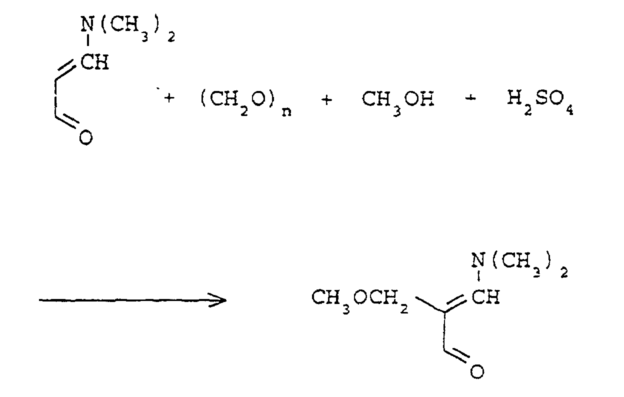

Concentrated sulfuric acid (1 mL) is slowly added to a solution of 3-(dimethylamino)-2-propenal (200 g, 2.01 mol) and paraformaldehyde (90 g, 3 mol) in methanol (1 L). The resultant solution is refluxed overnight, concentrated *in vacuo* to a volume of 200 mL, diluted with toluene and distilled until the vapor temperature is 105 °C. The solution is then concentrated *in vacuo* to give the title product as an orange oil (251.4 g, 87% yield).

### EXAMPLE 9

### Preparation of 3-Methoxy-2-(methoxymethyl)-2-propenal, (E)- and (Z)-

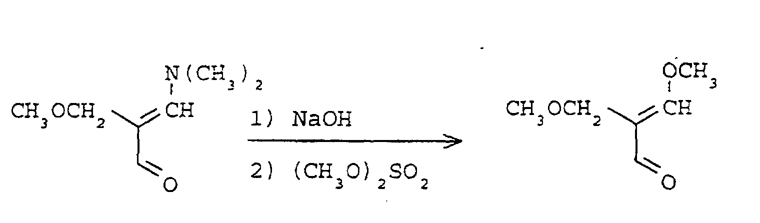

A solution of 3-(dimethylamino)-2-(methoxymethyl)-2-propenal, (E)- and (Z)- (53.06 g, 0.37 mol) and sodium hydroxide solution (29.7 g, 50%, 0.37 mol) in methanol (60 mL) is refluxed for 20 minutes and concentrated in *vacuo* to obtain a white solid. A solution of the solid in water (250 mL) is treated dropwise with dimethyl sulfate (46.75 g, 0.37 mol), stirred at room temperature for one hour and extracted with methylene chloride. The organic extract is dried over anhydrous sodium sulfate, concentrated *in vacuo* and distilled to give the title product as a colorless liquid (19.66 g, bp 80 °C/0.5 mm Hg, 41% yield).

### EXAMPLE 10

### Preparation of 5-(Methoxymethyl)-N-phenyl-2,3-pyridinedicarboximide

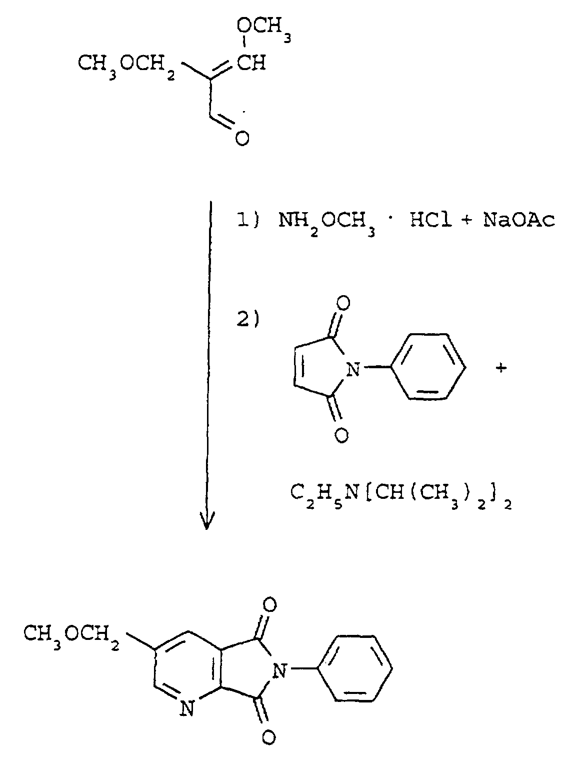

A solution of methoxyamine hydrochloride (1.7 g, 20 mmol) and sodium acetate (2.1 g, 25.6 mmol) in water (30 mL) is treated dropwise with 3-methoxy-2-(methoxymethyl)-2-propenal, (E)- and (Z)- (2.2 g, 16.9 mmol), stirred at room temperature for 30 minutes and extracted with methylene chloride. The organic extract is dried over anhydrous sodium sulfate and concentrated *in vacuo* to obtain the O-methyloxime of 3-methoxy-2-(methoxymethyl)-2-propen-1-one. A mixture of the resultant O-methyloxime of 3-methoxy-2-(methoxymethyl)-2-propen-1-one, N-phenylmaleimide (2.9 g, 16.8 mmol) and diisopropylethylamine (2.2 g, 17.0 mmol) in toluene (50 mL) is refluxed for 23 hours. During the reflux period, additional N-phenylmaleimide (2.9 g, 16.8 mmol) is added to the reaction mixture. The final reaction mixture is concentrated *in vacuo* to give the title product as a solid (0.36 g, 8% yield) having a 5-(methoxymethyl)-N-phenyl-2,3-pyridinedicarboximide to 5-methyl-N-phenyl-2,3-pyridinedicarboximide ratio of 50:1.

### EXAMPLE 11

### Preparation of 3-Ethoxy-2-methylacrolein dimethylhydrazone, (E) - and (Z)-

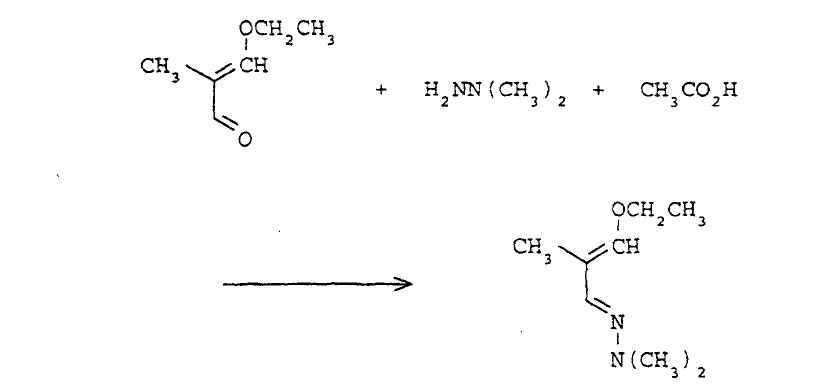

A mixture of 3-ethoxy-2-methyl-2-propenal, (E)- and (Z)- (4.0 g, 35 mmol), 1,1-dimethylhydrazine (2.73 g, 46 mmol) and acetic acid (0.04 g, 0.7 mmol) in diethyl ether is refluxed for one hour, cooled, washed sequentially with water and brine, dried over anhydrous magnesium sulfate, and concentrated *in vacuo* to give the title product as a yellow oil.

### EXAMPLE 12

### Preparation of 5-Methyl-N-phenyl-2,3-pyridine-dicarboximide from N-phenylmaleimide and 3-ethoxy-2-methylacrolein dimethylhydrazone, (E) - and (Z) -

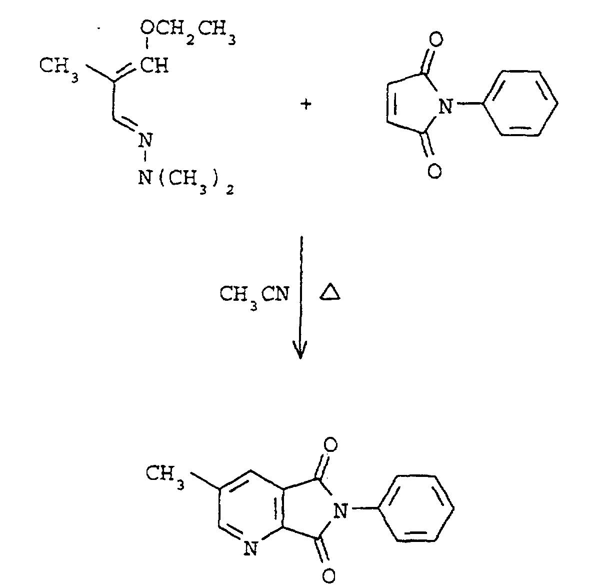

A solution of N-phenylmaleimide (1.1 g, 6.4 mmol) in acetonitrile is refluxed for 19 hours. During the reflux period, 3-ethoxy-2-methylacrolein dimethylhydrazone, (E)-and (Z)- (1.2 g, 7.6 mmol) is added portionwise to the reaction mixture. The final reaction mixture is then concentrated *in vacuo* to give the title product as a dark oil (0.23 g, 15% yield).

## Claims

1. A process for the preparation of a 2,3-pyridinedicarboximide having the structural formula I 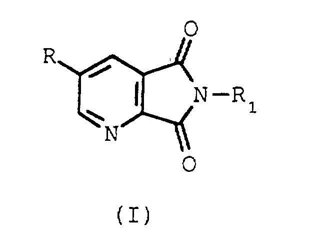 wherein
R is hydrogen, C₁-C₆alkyl or C₁-C₆alkoxymethyl;
R₁ is hydrogen, C₁-C₆alkyl, C(O)R₂,
phenyl optionally substituted with any combination of from one to four halogen, C₁-C₄alkyl, C₁-C₄alkoxy, nitro or cyano groups,
benzyl optionally substituted on the phenyl ring with any combination of from one to four halogen, C₁-C₄alkyl, C₁-C₄alkoxy, nitro or cyano groups, or 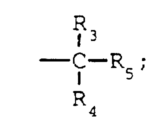
R₂ is C₁-C₆alkyl, benzyl or
phenyl optionally substituted with any combination of from one to four halogen, C₁-C₄alkyl, C₁-C₄alkoxy, nitro or cyano groups;
R₃ and R₄ are each independently C₁-C₄alkyl; and
R₅ is cyano or CONH₂,
which process comprises reacting an oxime or hydrazone having the structural formula II 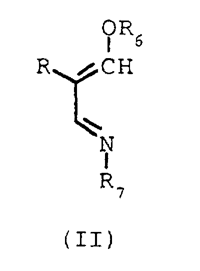 wherein
R is as described above;
R₆ is C₁-C₆alkyl;
R₇ is OR₈ or NR₉R₁₀;
R₈ is hydrogen, C₁-C₆alkyl, C(O)R₁₁,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups;
R₁₁ is C₁-C₆alkyl, OR₁₂, NR₁₂R₁₃, benzyl or
phenyl optionally substituted with any combination of from one to four halogen, C₁-C₄alkyl, C₁-C₄alkoxy, nitro or cyano groups;
R₁₂ and R₁₃ are each independently hydrogen, C₁-C₆alkyl, benzyl or
phenyl optionally substituted with any combination of from one to four halogen, C₁-C₄alkyl, C₁-C₄alkoxy, nitro or cyano groups; and
R₉ and R₁₀ are each independently hydrogen, C₁-C₆alkyl, benzyl or
phenyl optionally substituted with any combination of from one to four halogen, C₁-C₄alkyl, C₁-C₄alkoxy, nitro or cyano groups,
with a maleimide having the structural formula III 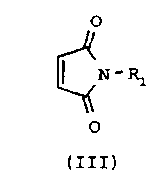 wherein R₁ is as described above.

2. The process according to Claim 1 wherein
R is hydrogen, C₁-C₄alkyl or C₁-C₄alkoxymethyl;
R₁ is hydrogen, C₁-C₄alkyl,
phenyl optionally substituted with any combination of from one to four halogen, C₁ -C₄alkyl, C₁-C₄alkoxy, nitro or cyano groups, or 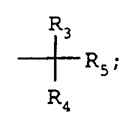
R₆ is C₁-C₄alkyl;
R₇ is OR₈; and
R₈ is hydrogen or C₁-C₆alkyl.

3. The process according to Claim 1 or Claim 2 wherein the formula II oxime or hydrazone is reacted with the formula III maleimide in the presence of a solvent selected from the group consisting of an aromatic hydrocarbon, a halogenated aromatic hydrocarbon, a polynuclear aromatic hydrocarbon, a glycol, an alkanoic acid, an alkanoic acid/water mixture, acetonitrile, an acetonitrile/water mixture, and mixtures thereof, and the boiling point of the solvent is at least 60°C.

4. The process according to any one of Claim 1 to 3 wherein the formula II oxime or hydrazone is reacted with the formula III maleimide at a temperature of above 20°C.

5. The process according to any one of Claim 1 to 4 further comprising a Lewis acid selected from the group consisting of aluminum chloride or titanium(IV) chloride.

6. The process according to any one of Claim 1 to 5 further comprising a base selected from the group consisting of a tri(C₂-C₄alkyl)amine, an alkali metal acetate and mixtures thereof when R is C₁-C₆alkoxymethyl.

7. A compound having the structural formula 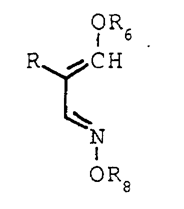 wherein
R is hydrogen, C₁-C₆alkyl or C₁-C₆alkoxymethyl;
R₆ is C₁-C₄alkyl;
R₈ is hydrogen, C₁-C₆alkyl, C(O)R₁₁,
phenyl optionally substituted with any combination of from one to four halogen, C₁-C₄alkyl, C₁-C₄alkoxy, nitro or cyano groups, or
benzyl optionally substituted on the phenyl ring with any combination of from one to four halogen, C₁-C₄alkyl, C₁-C₄alkoxy, nitro or cyano groups;
R₁₁ is C₁-C₆alkyl, OR₁₂, NR₁₂R₁₃, benzyl or
phenyl optionally substituted with any combination of from one to four halogen, C₁-C₄alkyl, C₁-C₄alkoxy, nitro or cyano groups; and
R₁₂ and R₁₃ are each independently hydrogen, C₁-C₆alkyl, benzyl or
phenyl optionally substituted with any combination of from one to four halogen, C₁-C₄alkyl, C₁-C₄alkoxy, nitro or cyano groups, and
the cis and trans isomers thereof.

8. The compound according to claim 7 wherein
R is hydrogen, C₁-C₄alkyl or C₁-C₄alkoxymethyl;
R₆ is C₁-C₄alkyl; and
R₈ is hydrogen or C₁-C₆alkyl.

9. The compound according to claim 7 selected from the group consisting of
the O-methyloxime of 3-ethoxy-2-methyl-2-propen-1-one;
the O-methyloxime of 3-methoxy-2-(methoxymethyl)-2-propen-1-one;
the oxime of 3-ethoxy-2-methyl-2-propen-1-one; and
the oxime of 3-methoxy-2-(methoxymethyl)-2-propen-1-one.

10. A process for the preparation of a herbicidal imidazolinone compound having the formula XII
wherein 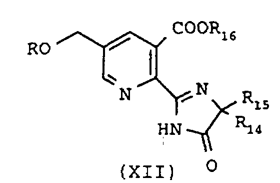
R is as defined in claim 1;
R₁₄ is C₁-C₄ alkyl;
R₁₅ is C₁-C₄ alkyl, C₃-C₆ cycloalkyl or R₁₄ and R₁₅ when taken together with the atom to which they are attached, represent a C₃-C₆ cycloalkyl group optionally substituted with methyl and
R₁₆ is hydrogen, diC₁-C₄alkylimino,
C₁-C₁₂ alkyl optionally substituted with one of the following groups: C₁-C₃ alkoxy, halogen, hydroxy, C₃-C₆ cycloalkyl, benzyloxy, furyl, phenyl, halophenyl, C₁-C₄ alkylphenyl, C₁-C₄ alkoxyphenyl, nitrophenyl, carboxyl, C₁-C₄ alkoxycarbonyl, cyano or tri-C₁-C₄ alkylammonium;
C₃-C₁₂ alkenyl optionally substituted with one of the following groups: C₁-C₃ alkoxy, phenyl, halogen or C₁-C₄ alkoxycarbonyl or with two C₁-C₃ alkoxy groups or two halogen groups;
C₃-C₆ cycloalkyl optionally substituted with one or two C₁-C₃ alkyl groups; or
a cation which process comprises:
(a) preparing a compound having the formula I 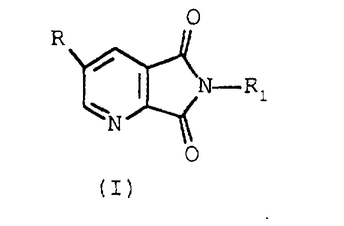 wherein R and R₁ are as defined in Claim 1 by a process as claimed in Claim 1; and
(b) converting the compound having formula I into the compound having the formula XII.

## Patentansprüche

1. Verfahren zur Herstellung eines 2,3-Pyridindicarboximids mit der Strukturformel I 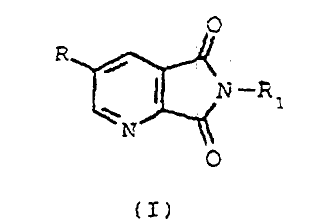 in welcher
R für Wasserstoff, C₁-C₆-Alkyl oder C₁-C₆ Alkoxymethyl steht;
R₁ für Wasserstoff, C₁-C₆-Alkyl, C(O)R₂, Phenyl, welches gegebenenfalls durch eine beliebige Kombination von ein bis vier Halogen-, C₁-C₄-Alkyl-, C₁-C₄-Alkoxy-, Nitro oder Cyanogruppen substituiert ist, Benzyl, welches gegebenenfalls am Phenylring durch eine beliebige Kombination von ein bis vier Halogen-, C₁-C₄-Alkyl-, C₁-C₄-Alkoxy-, Nitro- oder Cyanogruppen substituiert ist, oder 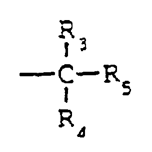 steht;
R₂ für C₁-C₆-Alkyl, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht;
R₃ und R₄ jeweils unabhängig voneinander für C₁-C₄-Alkyl stehen; und
R₅ für Cyano oder CONH₂ steht,
bei dem man ein Oxim oder Hydrazon mit der Strukturformel II 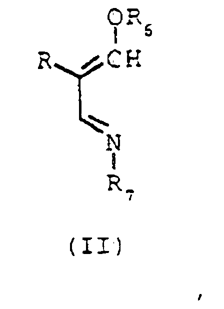
in welcher
R₁ wie oben beschrieben ist;
R₆ für C₁-C₆-Alkyl steht;
R₇ für OR₈ oder NR₉R₁₀ steht;
R₈ für Wasserstoff, C₁-C₆-Alkyl, C(O)R₁₁, Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, oder Benzyl, welches gegebenenfalls am Phenylring durch eine beliebige Kombination von ein bis vier Halogen-, C₁-C₄-Alkyl-, C₁-C₄-Alkoxy-, Nitro- oder Cyanogruppen substituiert ist, steht;
R₁₁ für C₁-C₆-Alkyl, OR₁₂, NR₁₂R₁₃, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht;
R₁₂ und R₁₃ jeweils unabhängig voneinander für Wasserstoff, C₁-C₆-Alkyl, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht; und
R₉ und R₁₀ jeweils unabhängig voneinander für Wasserstoff, C₁-C₆-Alkyl, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht, mit einem Maleinimid mit der Strukturformel III 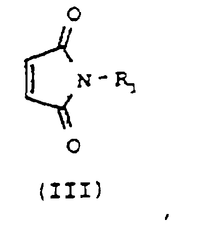 in welcher R₁ wie oben beschrieben ist, umsetzt.

2. Verfahren nach Anspruch 1, wobei
R für Wasserstoff, C₁-C₄-Alkyl oder C₁-C₄-Alkoxymethyl steht;
R₁ für Wasserstoff, C₁-C₄-Alkyl, Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, oder 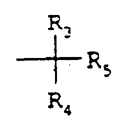 steht;
R₆ für C₁-C₄-Alkyl steht;
R₇ für OR₈ steht; und
R₈ für Wasserstoff oder C₁-C₆-Alkyl steht.

3. Verfahren nach Anspruch 1 oder 2, bei dem man das Oxim oder Hydrazon der Formel II in Gegenwart eines aus der aus einem aromatischen Kohlenwasserstoff, einem aromatischen Halogenkohlenwasserstoff, einem mehrkernigen aromatischen Kohlenwasserstoff, einem Glycol, einer Alkansäure, einer Alkansäure-Wasser-Mischung, Acetonitril, einer Acetonitril-Wasser-Mischung und Mischungen davon bestehenden Gruppe ausgewählten Lösungsmittels mit dem Maleinimid der Formel III umsetzt und der Siedepunkt des Lösungsmittels wenigstens 60°C beträgt.

4. Verfahren nach einem der Ansprüche 1 bis 3, bei dem man das Oxim oder Hydrazon der Formel II bei einer Temperatur oberhalb von 20°C mit dem Maleinimid der Formel III umsetzt.

5. Verfahren nach einem der Ansprüche 1 bis 4, weiterhin umfassend eine aus der aus Aluminiumchlorid und Titan(IV)chlorid bestehenden Gruppe ausgewählte Lewis-Säure.

6. Verfahren nach einem der Ansprüche 1 bis 5, weiterhin umfassend eine aus der aus einem Tri-(C₂-C₄-alkyl)amin, einem Alkaliacetat und Mischungen davon bestehenden Gruppe ausgewählte Base, wenn R für C₁-C₆-Alkoxymethyl steht.

7. Verbindungen mit der Strukturformel 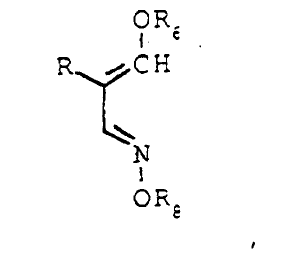 in welcher
R für Wasserstoff, C₁-C₆-Alkyl oder C₁-C₆-Alkoxymethyl steht;
R₆ für C₁-C₄-Alkyl steht;
R₈ für Wasserstoff, C₁-C₆-Alkyl, C(O)R₁₁,
Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, oder
Benzyl, welches gegebenenfalls am Phenylring durch eine beliebige Kombination von ein bis vier Halogen-, C₁-C₄-Alkyl-, C₁-C₄-Alkoxy-, Nitro- oder Cyanogruppen substituiert ist, steht;
R₁₁ für C₁-C₆-Alkyl, OR₁₂, NR₁₂R₁₃, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht;
R₁₂ und R₁₃ jeweils unabhängig voneinander für Wasserstoff, C₁-C₆-Alkyl, Benzyl oder Phenyl, welches gegebenenfalls durch eine beliebige Kombination von ein bis vier Halogen-, C₁-C₄-Alkyl-, C₁-C₄-Alkoxy-, Nitro- oder Cyanogruppen substituiert ist, steht, und
deren cis- und trans-Isomere.

8. Verbindungen nach Anspruch 7, wobei
R für Wasserstoff, C₁-C₄-Alkyl oder C₁-C₄-Alkoxymethyl steht;
R₆ für C₁-C₄-Alkyl steht; und
R₈ für Wasserstoff oder C₁-C₆-Alkyl steht.

9. Verbindungen nach Anspruch 7, ausgewählt aus der aus dem O-Methyloxim von 3-Ethoxy-2-methyl-2-propen-1-on;
dem O-Methyloxim von 3-Methoxy-2-(methoxy methyl)-2-propen-1-on;
dem Oxim von 3-Ethoxy-2-methyl-2-propen-1-on; und
dem Oxim von 3-Methoxy-2-(methoxymethyl)-2-propen-1-on
bestehenden Gruppe.

10. Verfahren zur Herstellung einer herbiziden Imidazolinonverbindung mit der Formel XII 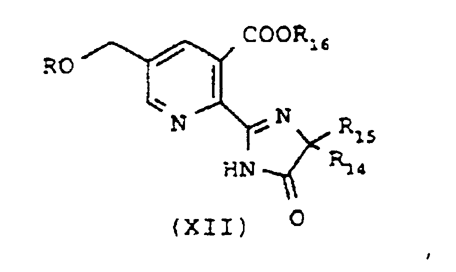 wobei
R wie in Anspruch 1 definiert ist;
R₁₄ für C₁-C₄-Alkyl steht;
R₁₅ für C₁-C₄-Alkyl oder C₃-C₆-Cycloalkyl steht oder R₁₄ und R₁₅ zusammen mit dem Atom, an das sie gebunden sind, für eine gegebenenfalls Methyl-substituierte C₃-C₆-Cycloalkylgruppe stehen, und
R₁₆ für Wasserstoff, Di-C₁-C₄-alkylimino, C₁-C₁₂-Alkyl, welches gegebenenfalls durch eine der folgenden Gruppen substituiert ist: C₁-C₃-Alkoxy, Halogen, Hydroxyl, C₃-C₆-Cycloalkyl, Benzyloxy, Furyl, Phenyl, Halogenphenyl, C₁-C₄-Alkylphenyl, C₁-C₄-Alkoxyphenyl, Nitrophenyl, Carboxyl, C₁-C₄-Alkoxycarbonyl, Cyano oder Tri-C₁-C₄-alkylammonium;
C₃-C₁₂-Alkenyl, welches gegebenenfalls durch eine der folgenden Gruppen substituiert ist: C₁-C₃-Alkoxy, Phenyl, Halogen oder C₁-C₄-Alkoxycarbonyl oder durch zwei C₁-C₃-Alkoxygruppen oder zwei Halogengruppen;
C₃-C₆-Cycloalkyl, welches gegebenenfalls durch ein oder zwei C₁-C₃-Alkylgruppen substituiert ist; oder
ein Kation steht,
bei dem man:
(a) eine Verbindung mit der Formel I 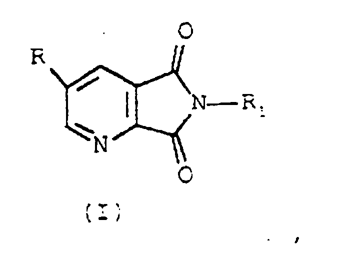 in welcher R und R₁ wie in Anspruch 1 definiert sind, durch ein Verfahren nach Anspruch 1 darstellt; und
(b) die Verbindung mit der Formel I in die Verbindung mit der Formel XII umwandelt.

## Revendications

1. Procédé pour la préparation d'un 2,3-pyridine-dicarboximide ayant la formule développée I 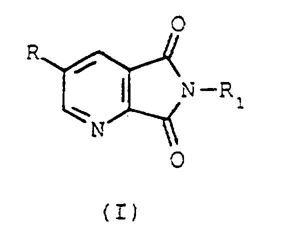 dans laquelle
R est l'hydrogène, un groupe alkyle en C₁-C₆ ou (alcoxy en C₁-C₆)méthyle ;
R₁ est l'hydrogène, un groupe alkyle en C₁-C₆, C(O)R₂,
phényle facultativement substitué par toute association d'un à quatre groupes halogéno, alkyle en C₁-C₄, alcoxy en C₁-C₄, nitro ou cyano,
benzyle facultativement substitué sur le noyau phényle par toute association d'un à quatre groupes halogéno, alkyle en C₁-C₄, alcoxy en C₁-C₄, nitro ou cyano, ou 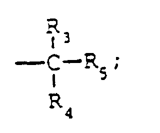
R₂ est un groupe alkyle en C₁-C₆, benzyle ou phényle facultativement substitué par toute association d'un à quatre groupes halogéno, alkyle en C₁-C₄, alcoxy en C₁-C₄, nitro ou cyano ;
R₃ et R₄ sont chacun indépendamment un groupe alkyle en C₁-C₄ ; et
R₅ est un groupe cyano ou CONH₂,
ce procédé comprenant la réaction d'une oxime ou hydrazone ayant la formule développée II 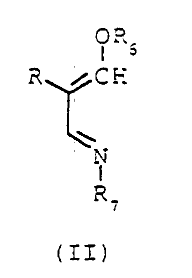 dans laquelle
R est tel que défini ci-dessus ;
R₆ est un groupe alkyle en C₁-C₆ ;
R₇ est OR₈ ou NR₉R₁₀ ;
R₈ est l'hydrogène, un groupe alkyle en C₁-C₆, C(O)R₁₁,
phényle facultativement substitué par toute association d'un à quatre groupes halogéno, alkyle en C₁-C₄, alcoxy en C₁-C₄, nitro ou cyano, ou
benzyle facultativement substitué sur le noyau phényle par toute association d'un à quatre groupes halogéno, alkyle en C₁-C₄, alcoxy en C₁-C₄, nitro ou cyano ;
R₁₁ est un groupe alkyle en C₁-C₆, OR₁₂, NR₁₂R₁₃, benzyle ou phényle facultativement substitué par toute association d'un à quatre groupes halogéno, alkyle en C₁-C₄, alcoxy en C₁-C₄, nitro ou cyano ;
R₁₂ et R₁₃ sont chacun indépendamment l'hydrogène, un groupe alkyle en C₁-C₆, benzyle ou
phényle facultativement substitué par toute association d'un à quatre groupes halogéno, alkyle en C₁-C₄, alcoxy en C₁-C₄, nitro ou cyano ; et
R₉ et R₁₀ sont chacun indépendamment l'hydrogène, un groupe alkyle en C₁-C₆, benzyle ou
phényle facultativement substitué par toute association d'un à quatre groupes halogéno, alkyle en C₁-C₄, alcoxy en C₁-C₄, nitro ou cyano,
avec un maléimide ayant la formule développée III 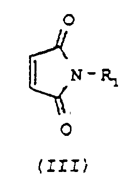 dans laquelle R₁ est tel que défini ci-dessus.

2. Procédé selon la revendication 1, dans lequel
R est l'hydrogène, un groupe alkyle en C₁-C₄ ou (alcoxy en C₁-C₄)méthyle ;
R₁ est l'hydrogène, un groupe alkyle en C₁-C₄,
phényle facultativement substitué par toute association d'un à quatre groupes halogéno, alkyle en C₁-C₄, alcoxy en C₁-C₄, nitro ou cyano, ou 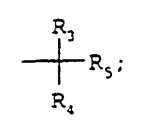
R₆ est un groupe alkyle en C₁-C₄ ;
R₇ est OR₈ ; et
R₈ est l'hydrogène ou un groupe alkyle en C₁-C₆.

3. Procédé selon la revendication 1 ou la revendication 2, dans lequel l'oxime ou l'hydrazone de formule II est amenée à réagir avec le maléimide de formule III en présence d'un solvant choisi dans la classe formée par un hydrocarbure aromatique, un hydrocarbure aromatique halogéné, un hydrocarbure aromatique polynucléaire, un glycol, un acide alcanoïque, un mélange acide alcanoïque/eau, l'acétonitrile, un mélange acétonitrile/eau, et leurs mélanges, et le point d'ébullition du solvant est d'au moins 60°C.

4. Procédé selon l'une quelconque des revendications 1 à 3, dans lequel l'oxime ou hydrazone de formule II est amenée à réagir avec le maléimide de formule III à une température supérieure à 20°C.

5. Procédé selon l'une quelconque des revendications 1 à 4, comprenant de plus un acide de Lewis choisi dans la classe formée par le chlorure d'aluminium ou le chlorure de titane(IV).

6. Procédé selon l'une quelconque des revendications 1 à 5, comprenant de plus une base choisie dans la classe formée par une tri(alkyle en C₂-C₄)amine, un acétate de métal alcalin et leurs mélanges lorsque R est un groupe (alcoxy en C₁-C₆)méthyle.

7. Composé ayant la formule développée 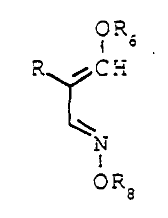 dans laquelle
R est l'hydrogène, un groupe alkyle en C₁-C₆ ou (alcoxy en C₁-C₆)méthyle ;
R₆ est un groupe alkyle en C₁-C₄ ;
R₈ est l'hydrogène, un groupe alkyle en C₁-C₆, C(O)R₁₁,
phényle facultativement substitué par toute association d'un à quatre groupes halogéno, alkyle en C₁-C₄, alcoxy en C₁-C₄, nitro ou cyano, ou
benzyle facultativement substitué sur le noyau phényle par toute association d'un à quatre groupes halogéno, alkyle en C₁-C₄, alcoxy en C₁-C₄, nitro ou cyano ;
R₁₁ est un groupe alkyle en C₁-C₆, OR₁₂, NR₁₂R₁₃, benzyle ou phényle facultativement substitué par toute association d'un à quatre groupes halogéno, alkyle en C₁-C₄, alcoxy en C₁-C₄, nitro ou cyano ; et
R₁₂ et R₁₃ sont chacun indépendamment l'hydrogène, un groupe alkyle en C₁-C₆, benzyle ou
phényle facultativement substitué par toute association d'un à quatre groupes halogéno, alkyle en C₁-C₄, alcoxy en C₁-C₄, nitro ou cyano,
et leurs isomères *cis* et *trans.*

8. Composé selon la revendication 7, dans lequel
R est l'hydrogène, un groupe alkyle en C₁-C₄ ou (alcoxy en C₁-C₄)méthyle ;
R₆ est un groupe alkyle en C₁-C₄ ; et
R₈ est l'hydrogène ou un groupe alkyle en C₁-C₆.

9. Composé selon la revendication 7, choisi dans la classe formée par
la O-méthyloxime de 3-éthoxy-2-méthyl-2-propène-1-one ;
la O-méthyloxime de 3-méthoxy-2-(méthoxyméthyl)-2-propène-1-one ;
l'oxime de 3-éthoxy-2-méthyl-2-propène-1-one; et
l'oxime de 3-méthoxy-2-(méthoxyméthyl)-2-propène-1-one.

10. Procédé pour la préparation d'une imidazolinone herbicide ayant la formule XII 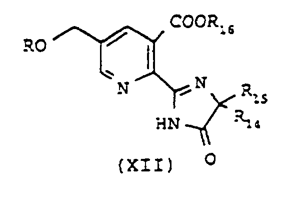 dans laquelle
R est tel que défini dans la revendication 1 ;
R₁₄ est un groupe alkyle en C₁-C₄ ;
R₁₅ est un groupe alkyle en C₁-C₄, cycloalkyle en C₃-C₆, ou bien R₁₄ et R₁₅ représentent, lorsqu'ils sont pris avec l'atome auquel ils sont fixés, un groupe cycloalkyle en C₃-C₆ facultativement substitué par un groupe méthyle et
R₁₆ est l'hydrogène, un groupe di(alkyle en C₁-C₄)imino,
alkyle en C₁-C₁₂ facultativement substitué par l'un des groupes suivants : alcoxy en C₁-C₃, halogéno, hydroxyle, cycloalkyle en C₃-C₆, benzyloxy, furyle, phényle, halogénophényle, (alkyle en C₁-C₄)phényle, (alcoxy en C₁-C₄)phényle, nitrophényle, carboxyle, (alcoxy en C₁-C₄)carbonyle, cyano ou tri(alkyle en C₁-C₄) ammonium ;
alcényle en C₃-C₁₂ facultativement substitué par l'un des groupes suivants : alcoxy en C₁-C₃, phényle, halogéno ou (alcoxy en C₁-C₄)carbonyle, ou par deux groupes alcoxy en C₁-C₃ ou deux groupes halogéno ;
cycloalkyle en C₃-C₆ facultativement substitué par un ou deux groupes alkyle en C₁-C₃ ; ou
un cation
ce procédé consistant à :
(a) préparer un composé ayant la formule I 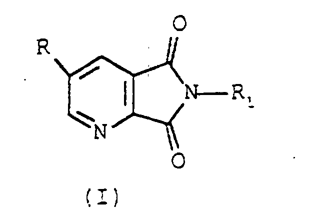 dans laquelle R et R₁ sont tels que définis dans la revendication 1, par un procédé selon la revendication 1 ; et
(b) convertir le composé ayant la formule I en le composé ayant la formule XII.
